# FASCICULE DE BREVET EUROPEEN

(11) **EP 2 501 713 B1**
(45) Date de publication et mention de la délivrance du brevet: **27.07.2016**
(21) Numéro de dépôt: 10787915.7
(22) Date de dépôt: 18.11.2010
(51) Int. Cl.: C07K 1/36, C07K 14/745, C07K 14/75

(54) **PROCEDE DE TRAITEMENT DU PLASMA SANGUIN COMPRENANT UNE ETAPE DE LAVAGE PAR DISPERSION**
VERFAHREN ZUR BEHANDLUNG VON BLUTPLASMA MIT EINEM WASCHSCHRITT MITTELS DISPERSION
METHOD FOR TREATING BLOOD PLASMA INCLUDING A STEP OF WASHING BY MEANS OF DISPERSION

(30) Priorité: 18.11.2009 FR 0958145
(43) Date de publication de la demande: 26.09.2012
(73) Titulaire: Laboratoire Français du Fractionnement et des Biotechnologies, 91940 Les Ulis (FR)
(72) Inventeur: TRUSGNICH, Thierry, F-94370 Sucy En Brie (FR)
(74) Mandataire: Cabinet Plasseraud
(86) Numéro de dépôt international: PCT/IB2010/055263
(87) Numéro de publication internationale: WO 2011/061705

(56) Documents cités:
- EP-A1- 1 155 706
- EP-A1- 1 739 093
- WO-A1-01/48016

## Description

### Domaine technique

La présente invention concerne un procédé de traitement du plasma sanguin comprenant les étapes de précipitation éthanolique du plasma ou d'une fraction de plasma, de récupération du précipité, de lavage dudit précipité, de récupération d'une pâte plasmatique lavée, de solubilisation de ladite pâte plasmatique lavée.

### Etat de la technique

Le fibrinogène est une protéine essentielle de la coagulation sanguine, car sa polymérisation en fibrine insoluble formée au terme de la cascade des réactions qui gouvernent la coagulation, aboutit à la formation d'un caillot obturant la brèche vasculaire, responsable du saignement. La mise en place du caillot est ainsi essentielle pour assurer l'arrêt du saignement. De plus, la fibrine formée au niveau de la plaie constitue un réseau fibrillaire qui assure la réparation tissulaire (cicatrisation).

Des déficiences congénitales en fibrinogène peuvent conduire à de graves pathologies. Pour soigner ces déficiences, il est nécessaire de disposer de concentrés de fibrinogène pouvant être administrés à des patients en traitement. D'autres pathologies peuvent également être soignées par des apports de fibrinogène, notamment en cas de pertes massives de sang (chirurgie, traumatismes etc.), ou à la suite d'une coagulopathie de consommation décompensée (CIVD).

Par ailleurs, les colles biologiques activables par la thrombine renfermant du fibrinogène, en tant que constituant majeur, et du Facteur XIII (FXIII), sont efficacement utilisées pour la réparation tissulaire lors d'usages cliniques, tels les greffes de peau, les sutures nerveuses ou artérielles, comme décrit, par exemple, dans les brevets EP 0 305 243, FR 2 448 900 et FR 2 448 901.

La présence de Facteur XIII ou transglutaminase dans ces produits contribue à stabiliser la fibrine par la création de liaisons covalentes intercaténaires qui la rendent insoluble. Dans certains cas, ces produits sont obtenus selon des procédés de production du fibrinogène assez complexes qui nécessitent un apport exogène de Facteur XIII purifié afin qu'ils puissent remplir leur fonction thérapeutique.

Par conséquent, la mise à disposition de concentrés de fibrinogène, de colles biologiques, et de Facteur XIII, notamment à des fins thérapeutiques, requiert des techniques de purification conduisant à ces produits non seulement suffisamment purifiés de contaminants de nature diverse; tels que les protéines accompagnantes ou coprécipitées, les anticorps ou les protéases, mais de surcroît sécurisés sur le plan viral.

L'isolement de fractions enrichies en fibrinogène, contenant éventuellement du FXIII, à partir du plasma est connu et a été décrit initialement par les travaux de Cohn et Nitschmann (Cohn et al, J. Am. Chem. Soc., 68, 459, 1946 et Kistler et al, Vox Sang., 7, 1962, 414-424). Des méthodes plus récentes associent des techniques de précipitation de différentes sources de plasma et des techniques de filtration, de chromatographie, d'inactivation virale etc. On peut citer, à titre d'exemple, les brevets et demandes de brevets EP 0 359 593, US 5 099 003, EP 0 305 243, FR 2 448 900 et FR 2 448 901.

Il apparaît toutefois que la plupart des procédés connus dans l'état de la technique impliquent l'utilisation de chaînes de production distinctes et donc de méthodes différentes pour produire des concentrés ou des compositions de fibrinogène, de colle biologique ou enrichis en fibrinogène et contenant d'autres protéines accompagnantes comme le FXIII, le Facteur VIII, la fibronectine, le facteur von Willebrand etc. La plupart des procédés connus sont donc peu adaptés à une mise en oeuvre à l'échelle industrielle, en particulier lorsqu'un besoin conjoint en colle biologique, en fibrinogène et en facteur XIII existe. La complexité de mise en oeuvre de ces procédés de purification à l'échelle industrielle est encore augmentée lorsque les protéines d'intérêt sont destinées à un usage thérapeutique et qu'elles doivent être soumises à des traitements d'inactivation et/ou d'élimination des virus et autres contaminants indésirables, comme par exemple le prion.

La demande de brevet EP 1 739 093 décrit un procédé unique permettant de séparer les protéines fibrinogène, Facteur XIII et colle biologique d'une fraction plasmatique solubilisée comprenant du fibrinogène et du Facteur XIII, et conduisant à la préparation de concentrés lyophilisés desdites protéines. Le procédé décrit dans la demande EP 1 739 093 comprend en particulier les étapes de purification chromatographique d'une fraction plasmatique solubilisée sur un échangeur d'anions de type base faible dans des conditions permettant la rétention de la colle biologique, d'élution spécifique de cette colle, de séparation du FXIII à partir du fibrinogène par ajout d'au moins un agent chimique précipitant du FXIII, de récupération de la solution résultante de surnageant de fibrinogène purifié et de diafiltration des solutions de fibrinogène, de colle biologique et de FXIII remis en solution, suivie d'une lyophilisation desdites solutions. Le procédé décrit dans la demande de brevet EP 1 739 093 peut également comprendre au moins une étape de traitement d'inactivation virale et/ou d'élimination de virus et de contaminants, le traitement étant choisi parmi le traitement chimique d'inactivation virale, la nanofiltration et le traitement thermique d'inactivation virale à sec. Toutefois, les techniques de remise en suspension mises en oeuvre dans les procédés connus dans l'état de la technique ne sont pas totalement satisfaisantes. En effet, dans ces procédés connus dans l'état de la technique, le phénomène de cisaillement qui se produit lors de la remise en suspension peut engendrer une dégradation des protéines d'intérêt. Il existe donc un fort besoin pour la mise en oeuvre d'améliorations techniques susceptibles de diminuer la dégradation des protéines d'intérêt et augmenter le degré de pureté des protéines extraites.

### Résumé de l'invention

La Demanderesse a découvert de manière surprenante que la mise en oeuvre d'une étape de lavage par dispersion au cours de la phase préliminaire de préparation de la fraction plasmatique solubilisée utilisée en tant que matière première dans le cadre du procédé par exemple de la demande EP 1 739 093, permet d'améliorer de manière significative la qualité du lavage par rapport aux procédés de l'état de la technique, qui ne décrivent qu'une étape de lavage par remise en suspension. Il en ressort que la mise en oeuvre d'une étape de lavage par dispersion conduit à une augmentation du degré de pureté du fibrinogène dans la fraction plasmatique solubilisée, et, par suite, dans les concentrés de fibrinogène et de colle biologique résultant de la mise en oeuvre du procédé par exemple de la demande EP 1 739 093.

Les bénéfices obtenus par l'invention ne sont pas limités au seul procédé objet de la demande EP 1 739 093, mais seront obtenus avec les procédés utilisant la fraction éthanolique (fraction I de Cohn).

La présente demande décrit par conséquent un procédé de traitement du plasma sanguin comprenant les étapes de :
a) précipitation éthanolique du plasma ou d'une fraction de plasma ;
b) récupération du précipité formé à l'étape a);
c) lavage dudit précipité par dispersion ;
d) récupération d'une pâte plasmatique lavée ; et
e) solubilisation de ladite pâte plasmatique lavée.

Le lavage par dispersion de l'étape c) est, de préférence, réalisé en utilisant un disperseur de type rotor/stator. Avantageusement, le disperseur utilisé est un disperseur en ligne, le précipité de l'étape b) étant alimenté à l'entrée axiale du disperseur et le produit lavé étant refoulé par la sortie radiale du disperseur.

Le procédé de la demande peut également comprendre les étapes additionnelles de :
f) traitement de ladite pâte plasmatique solubilisée par l'hydroxyde d'aluminium et/ou traitement de ladite pâte plasmatique solubilisée à basse température ; et
g) obtention d'une fraction plasmatique soluble par filtration clarifiante et/ou stérilisante du surnageant résultant de l'étape f).

Le procédé tel que décrit peut enfin comporter des étapes supplémentaires de :
h) purification chromatographique de la fraction plasmatique soluble de l'étape g) sur une résine de type échangeur d'anions de type base faible préalablement équilibré par un tampon de force ionique prédéterminée de pH basique, dans des conditions permettant la rétention de la colle biologique, ladite colle biologique étant éluée par augmentation de la force ionique dudit tampon ;
i) précipitation du FXIII à partir de tout ou partie de l'éluat de colle biologique obtenu à l'étape h) par ajout d'au moins un agent chimique précipitant le FXIII ;
j) récupération d'une solution de fibrinogène purifié correspondant au surnageant de l'étape i) ; et
k) diafiltration des solutions de fibrinogène, et/ou de colle biologique et/ou de FXIII remis en solution, suivie d'une lyophilisation desdites solutions.

Ainsi, le procédé découvert par la Demanderesse, comprenant la mise en oeuvre d'une étape de lavage par dispersion, permet ainsi d'obtenir des concentrés de fibrinogène, de Facteur XIII et/ou de colle biologique lyophilisés, hautement purifiés, sensiblement dépourvus de protéines co-purifiées et de contaminants indésirables. En effet, l'étape de lavage par dispersion conduit à un meilleur lavage de la fraction plasmatique initiale comprenant les protéines d'intérêt, et en particulier le fibrinogène, et conduit à une amélioration significative du degré de pureté de ces composés au cours des étapes ultérieures du procédé, par rapport aux lavages par simple remise en suspension décrits dans l'état de la technique.

La mise en oeuvre d'une étape de lavage par dispersion conduit donc à une pureté plus élevée, dans un procédé simple, rapide et peu coûteux, et assure une rentabilisation améliorée de la matière première (plasma ou fraction de plasma) à l'échelle industrielle. Il en résulte une optimisation significative de la production de colle biologique, de Facteur XIII et/ou de fibrinogène, tout en réduisant les coûts de production qui y sont associés.

Le procédé de l'invention permet en outre d'obtenir des concentrés de protéines lyophilisées et hautement purifiées, à partir d'une matière première plasmatique contenant préférablement du fibrinogène et du Facteur XIII, tout en restant compatible avec au moins un traitement d'inactivation et/ou d'élimination des virus et d'autres contaminants indésirables (tels que des polymères, des agrégats ou des prions).

### Brève description des dessins

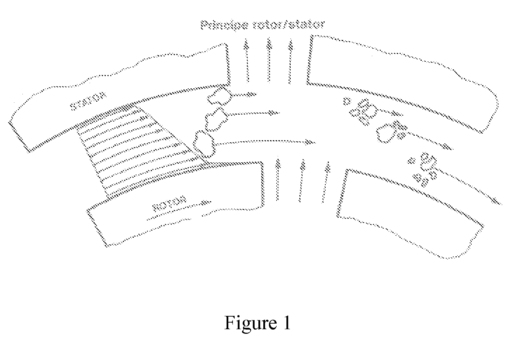

La figure unique est une représentation schématique d'un disperseur rotor/stator.

### Description détaillée de modes de réalisation de l'invention

La présente invention concerne par conséquent un procédé de traitement du plasma sanguin comprenant les étapes de :
a) précipitation éthanolique du plasma ou d'une fraction de plasma;
b) récupération du précipité formé à l'étape a);
c) lavage dudit précipité par dispersion en utilisant un disperseur de type rotor/stator ;
d) récupération d'une pâte plasmatique lavée ;
e) solubilisation de ladite pâte plasmatique lavée ;

Le procédé de l'invention peut être mis en oeuvre à partir de sources variées de matière première plasmatique, et en particulier à partir de sources contenant du fibrinogène et du Facteur XIII. Ce procédé peut ainsi être mis en oeuvre directement à partir du plasma en utilisant la méthode de fractionnement de Cohn, utilisant de l'alcool froid (Cohn et al, J. Am. Chem. Soc., 68, 459, 1946 et Kistler et al, Vox Sang., 7, 1962, 414-424).

Avantageusement, les étapes b) et d) du procédé de l'invention sont réalisées par centrifugation.

Au sens de la présente invention, le terme « dispersion » désigne la séparation des particules de précipité éthanolique et leur répartition homogène ou sensiblement homogène dans le milieu constitué par le tampon de lavage. Cette dispersion se distingue des méthodes de remise en suspension décrites dans l'état de la technique en particulier en ce que ces dernières ne permettent pas de répartir les particules de manière homogène.

Un disperseur de type rotor/stator comprend généralement au moins un rotor et au moins un stator dentés, formant un ensemble à grande vitesse de cisaillement. Le produit (ici, le précipité éthanolique mélangé au tampon de lavage) pénètre dans la zone de dispersion entre les fentes du rotor denté et ressort par les fentes de la couronne du stator, l'effet de pompage étant assuré par la machine elle-même. La couronne intérieure du rotor augmente la vitesse de passage du produit, la couronne stator le freine brusquement, favorisant la dispersion fine du produit. Les fortes turbulences dans la fente de cisaillement broient et dispersent avec efficacité les particules solides dans une répartition homogène. La géométrie du rotor et du stator peut être adaptée aux conditions de dispersion du produit. Un tel disperseur est représenté dans la figure unique.

Le disperseur, notamment rotor/stator et en particulier en ligne, permet donc d'obtenir une solution homogène ou sensiblement homogène, avec une dispersion très fine, homogène ou sensiblement homogène du précipité éthanolique dans le tampon de lavage, et contribue de ce fait à l'élimination d'une proportion plus importante de contaminants protéiques.

Si l'utilisation de disperseurs de type rotor/stator est connue dans l'état de la technique pour la préparation d'émulsions, pour la mise en suspension de pigments, pour l'incorporation de détergents dans une solution, pour la fabrication rapide de solutions colloïdales et pour l'homogénéisation de poudres et autres produits similaires, il convient toutefois de noter que l'utilisation de tels disperseurs n'est pas décrite dans le cadre de procédés de purification de protéines plasmatiques.

Il est en effet connu que l'utilisation d'un disperseur provoque un échauffement de la solution ou de la suspension préparée, et qu'un tel disperseur ne peut pas être utilisé dans une cuve thermostatée. Il paraissait par conséquent vraisemblable, à la date de la présente invention, de considérer que l'utilisation d'un disperseur dans le cadre de procédés de préparation de produits plasmatiques conduirait à une augmentation de la température, provoquant de ce fait la dégradation des protéines plasmatiques.

Or, la Demanderesse a observé de manière surprenante que l'utilisation d'un disperseur réduit le temps nécessaire à la dispersion et diminue en réalité l'échauffement de cette dernière. Ainsi, contrairement au préjugé technique en vigueur, et de manière surprenante, l'utilisation d'un disperseur se montre particulièrement appropriée dans le cadre d'un procédé de purification de protéines plasmatiques, dans la mesure où il n'engendre pas de dégradation du produit.

Par ailleurs, la Demanderesse a également observé que la mise en oeuvre d'un disperseur conduit à une diminution significative de la quantité de protéines contaminantes.

Dans un mode de réalisation préféré, l'étape de lavage par dispersion de l'étape c) du procédé de l'invention est réalisée en utilisant un disperseur en ligne. Dans ce cas particulier, le disperseur de type rotor/stator, est monté sur la même ligne de production que les autres éléments industriels requis pour mettre en oeuvre le procédé de purification. Le précipité de l'étape b) se trouve donc fourni, préférablement avec le tampon de lavage, à l'entrée axiale du disperseur en ligne et le produit lavé, correspondant à une dispersion sensiblement homogène du précipité éthanolique dans le tampon de lavage, est rejeté à la sortie radiale du disperseur en ligne.

L'utilisation d'un disperseur en ligne offre en outre toutes les garanties d'aseptie puisque la machine est conçue pour un démontage simple et un nettoyage facile et peut être intégré dans un NEP (nettoyage en place).

Comme discuté ci-dessus, le lavage du précipité éthanolique par un disperseur lors de l'étape c) du procédé de l'invention conduit à l'obtention d'une suspension homogène ou sensiblement homogène (un homogénat) du précipité éthanolique dans le tampon de lavage utilisé. Cette suspension homogène ou sensiblement homogène peut être caractérisée par un contrôle visuel permettant de vérifier l'absence d'agglomérat dans la suspension.

Dans un mode de réalisation particulier, l'étape c) de la présente invention est réalisée en utilisant une cuve équipée avec un mobile d'agitation de type pale défloculeuse. Ce mobile d'agitation permet de réaliser la dispersion primaire, qui aboutit à une pâte défloculée mais non homogène. Le mobile d'agitation est couplé à un système de dispersion en ligne, qui permet de réaliser la dispersion secondaire conduisant à une suspension fine et homogène. Dans un mode de réalisation préféré, la récupération du précipité effectuée à l'étape b) et/ou la récupération de la pâte plasmatique effectuée à l'étape d) sont réalisées par centrifugation. Préférablement, la récupération de la pâte plasmatique à l'étape d) est effectuée par la mise en oeuvre d'une centrifugeuse à injection. Dans ce cas particulier, le précipité lavé par dispersion de l'étape c) est introduit dans une centrifugeuse industrielle au moyen d'injecteurs dont le diamètre est calibré en fonction de la taille des particules en suspension. Dans un mode de réalisation préféré, la centrifugeuse à injection est une centrifugeuse de type « Sharpless AS 16» «ou westfalia separator », possédant des injecteurs spécifiques calibrés en fonction du séparateur utilisé (2 à 4 mm).

Le lavage par dispersion de l'étape c) permet d'obtenir une suspension comprenant de très fines particules de précipité éthanolique dans le tampon de lavage. Il en résulte que la suspension sensiblement homogène formée se révèle particulièrement bien adaptée à l'utilisation d'une centrifugeuse à injecteurs, dans la mesure où elle prévient avantageusement le colmatage des injecteurs de la centrifugeuse.

Dans un mode de réalisation préféré, la solubilisation de la pâte plasmatique est effectuée dans un tampon comprenant du chlorure de sodium 0.06-0.18 M, du citrate trisodique 0.005-0.02 M et de l'arginine 0.02-0.08 M, à pH 7.3 à 7.5.

Dans un mode de réalisation préféré, le précipité formé par la précipitation éthanolique est lavé par dispersion avec un tampon constitué d'un mélange de glycine 0.5-1.5 M, de citrate trisodique 0.01-0.1 M et d'éthanol 5.0-8.0% (v/v), à un pH de 6.7-6.9.

Dans un mode de réalisation préféré, le procédé de l'invention comprend les étapes additionnelles de :
f) traitement de ladite pâte plasmatique solubilisée par l'hydroxyde d'aluminium et/ou traitement de ladite pâte plasmatique solubilisée à basse température ;
g) obtention d'une fraction plasmatique soluble par filtration clarifiante et/ou stérilisante du surnageant résultant de l'étape f).

L'ajout d'hydroxyde d'aluminium lors de l'étape f) assure en particulier l'élimination des protéines indésirables, telles que les Facteurs II (FII), VII (FVII), IX (FIX) et X (FX). Le surnageant résultant du traitement de la pâte plasmatique solubilisée, aussi désigné « fraction plasmatique soluble » dans la présente demande, est purifié par au moins une filtration clarifiante et/ou stérilisante. La fraction plasmatique soluble peut également subir une filtration en profondeur dans le but de permettre la rétention de l'hydroxyde d'aluminium.

La filtration clarifiante, permet d'éliminer les particules contaminantes insolubles. Les filtrations clarifiante et stérilisante sont généralement mises en oeuvre par l'utilisation de filtres, par exemple, de 0,8 à 0,1 µm. Au moins une filtration clarifiante est avantageusement réalisée sous la forme d'une filtration sur filtres en fibres de cellulose de 0,65 µm Dans un mode de réalisation préféré, l'au moins une filtration stérilisante est effectuée sur filtres de 0,2 µm.

La fraction plasmatique soluble obtenue à l'issue de l'étape g) peut être utilisée directement ou peut, lorsque cela s'avère nécessaire, être congelée dans l'attente de la mise en oeuvre d'éventuelles étapes complémentaires de purification.

La mise en oeuvre du procédé de traitement de plasma sanguin comprenant une étape de lavage par dispersion permet avantageusement d'obtenir un degré de pureté du fibrinogène dans la fraction plasmatique soluble d'au moins 80%, de préférence d'au moins 85%, plus préférablement d'au moins 90%.

L'utilisation d'un système de dispersion en ligne lors de l'étape de lavage améliore en particulier l'enrichissement en fibrinogène au stade de la fraction plasmatique soluble. Cette technologie permet d'obtenir des résultats conformes et reproductibles en termes d'homogénéité de la suspension lors de la dispersion en ligne et d'enrichissement en fibrinogène lors de cette étape de lavage et, par suite, dans les étapes ultérieures, sans que la protéine d'intérêt subisse une dégradation.

Dans un mode de réalisation préféré, le procédé de traitement du plasma sanguin de l'invention comprend les étapes additionnelles de:
h) purification chromatographique de la fraction plasmatique soluble de l'étape g) sur une résine de type échangeur d'anions de type base faible préalablement équilibré par un tampon de force ionique prédéterminée de pH basique, dans des conditions permettant la rétention de la colle biologique, ladite colle biologique étant éluée par augmentation de la force ionique dudit tampon ;
i) précipitation du FXIII à partir de tout ou partie de l'éluat de colle biologique par ajout d'au moins un agent chimique précipitant le FXIII ;
j) récupération d'une solution de fibrinogène purifié correspondant au surnageant de l'étape i) ; et
k) diafiltration des solutions de fibrinogène, et/ou de colle biologique et/ou de FXIII remis en solution, suivie d'une lyophilisation desdites solutions.

La purification chromatographique de la fraction plasmatique soluble (étape h)) peut être effectuée sur toute matrice à base de polymère naturel ou synthétique, résine ou gel, sur laquelle sont greffés des groupements échangeurs d'anions de type base faible, tel que le DEAE. Les supports chromatographiques classiques de ce type sont disponibles sous les appellations DEAE-Sepharose CL-6B, DEAE-Trisacryl LS, Fractogel TSK-DEAE 650 M ou S, DEAE-Macroprep (Bio-Rad, France) etc.

Le tampon d'équilibrage de l'échangeur d'anions présente une force ionique prédéterminée et doit être à un pH basique. La force ionique est typiquement inférieure à la valeur de 0,2 et, de préférence, est située dans la plage de valeurs allant de 0,06 à 0,2, en particulier, de 0,08 à 0,15.

Celle-ci est de préférence ajustée par ajout de sels inorganiques de métaux alcalins ou alcalino-terreux ou leur mélange, de façon très préférée de sels inorganiques de métaux alcalins, en particulier de chlorure de sodium.

La valeur maximale du pH du tampon d'équilibrage est choisie de façon à éviter toute dénaturation des produits considérés, à savoir environ 10.

Avantageusement, le pH est situé dans la plage de valeurs supérieures à 7 jusqu'à 9, de préférence de 7,5 à 8,2.

A titre d'exemple, ce tampon est composé de chlorure de sodium, à un pH de 7,9-8,1, dont la concentration est de 0,06 M, et peut très préférentiellement comprendre en plus du citrate trisodique, à une concentration préférée de 0,011 M. On peut également utiliser tout autre tampon, à base de chlorure de sodium ou de sels inorganiques de métaux alcalins ou alcalino-terreux, comprenant d'autres composés biologiquement compatibles et non dénaturants pour les produits d'intérêt.

Lorsque la fraction plasmatique soluble a été appliquée sur l'échangeur d'anions, les conditions opératoires sont telles que la colle biologique est retenue sur le support.

Dans un mode de réalisation préféré, le procédé de l'invention peut comprendre, préalablement à l'étape d'élution de la colle biologique, une étape de lavage, avec ledit tampon d'équilibrage, de l'échangeur d'anions jusqu'à l'élimination des protéines et des contaminants non retenus. Cette étape de lavage permet, par percolation du tampon de lavage sur le support, le passage dans le filtrat des protéines présentes dans la solution contenant du fibrinogène, telles les immunoglobulines G (IgG), A (IgA) et M (IgM) et l'albumine, et des contaminants non retenus ou faiblement retenus par l'échangeur, comme les agents chimiques d'inactivation virale. La durée du lavage est déterminée par mesure de la densité optique (DO) du filtrat à la longueur d'onde de 280 nm. En effet, une valeur de DO correspondant à celle de la ligne de base est une bonne indication de l'élimination effective des composés cités ci-dessus.

Dans un mode de réalisation préféré, le tampon de lavage utilisé correspond au tampon d'équilibrage de la colonne et est préférablement constitué de chlorure de sodium 0.02-0.1 M et de citrate trisodique 0.005-0.02 M, à pH 7.8-8.2.

Après retour à la ligne de base, l'élution de la colle biologique est effectuée par augmentation de la force ionique du tampon d'équilibrage ou de lavage, dont le pH est de préférence fixé à 7,4-7,6. La valeur de cette force ionique est choisie de façon à obtenir l'élution efficace de la colle biologique tout en veillant à ce que cette valeur n'altère pas les propriétés du produit considéré.

Avantageusement, la valeur de la force ionique du tampon d'élution est comprise entre 0,5 et 1,3, en particulier entre 0,9 et 1,1. Cette augmentation de la force ionique est effectuée par ajout de tout sel ou mélange de sels définis ci-dessus, notamment de chlorure de sodium. Le tampon d'élution peut en outre contenir d'autres excipients, tel qu'un mélange de constituants, dénommé mélange A, comprenant le citrate trisodique (10 à 12 g/1), la lysine (1 à 5 g/1), la glycine (1 à 5 g/1), le sel tris (2 à 5 g/l), l'arginine (25 à 50 g/1) et l'isoleucine (5 à 15 g/1).

La concentration en protéines dans l'éluat est généralement de l'ordre de 4 g/1.

Au moins une partie de la quantité récoltée de l'éluat de colle biologique peut ensuite être soumise à un traitement destiné à séparer le FXIII accompagnant le fibrinogène.

Cette séparation est réalisée en faisant précipiter le FXIII par ajout dans l'éluat de l'étape h) d'un agent chimique précipitant, pouvant se présenter sous la forme d'une solution aqueuse, à une concentration appropriée pour obtenir la précipitation du facteur XIII.

De manière préférée, l'agent chimique précipitant est une solution aqueuse à base de sels de citrate 1 M, tels que le citrate de sodium et, en particulier, le citrate trisodique.

Le précipité de FXIII formé est ensuite séparé du surnageant très enrichi en fibrinogène. Dans un mode de réalisation préféré, le précipité de FXIII peut être récupéré par filtration sur des filtres de 5 µm

Le précipité de FXIII ainsi récupéré est ensuite remis en solution, de préférence dans de l'eau ou un tampon. Dans un mode de réalisation préféré, le précipité de FXIII est dissous dans un tampon comprenant un mélange de 10 à 12 g/l de citrate trisodique, de 1 à 5 g/l de lysine, de 1 à 5 g/l de glycine, de 2 à 5 g/l de Tris, de 25 à 50 g/l d'arginine et 5 à 15 g/l d'isoleucine, ajusté à un pH compris entre 6,9 et 7,1, de sorte que la concentration en Facteur XIII corresponde à une activité environ 100 fois supérieure à celle du plasma normal.

A ce titre, le précipité de FXIII peut par exemple être repris de façon que sa concentration soit d'environ 1 à 5 g de protéines totales/1.

Selon l'invention, les solutions de colle biologique (c'est-à-dire l'éluat de colle biologique résultant de l'étape h)) et de fibrinogène (surnageant enrichi en fibrinogène) peuvent avantageusement être concentrées par ultrafiltration, jusqu'à des teneurs typiquement comprises entre 15 et 25 g de protéines totales/1, déterminées par des mesures classiques connues de l'homme du métier.

Les trois solutions de fibrinogène, de Facteur XIII et de colle biologique obtenues, éventuellement concentrées, sont ensuite soumises à une étape de diafiltration.

L'étape de diafiltration permet d'éliminer l'excès éventuel, d'une part, de sel inorganique utilisé pour obtenir des solutions ayant une force ionique d'au plus 0,2 M, et, d'autre part, d'agent précipitant présent dans le précipité remis en solution. Il est à noter qu'une présence importante de sel inorganique, nécessaire à l'élution de la colle biologique, pourrait avoir une influence néfaste sur l'efficacité du processus de lyophilisation et de l'inactivation virale par chauffage thermique à sec ainsi que sur la capacité de rétention des virus par un nanofiltre approprié.

L'étape de diafiltration peut également être utilisée pour incorporer des excipients adéquats, tels que des stabilisants et/ou des protecteurs, destinés à autoriser, d'une part, un chauffage à sec du fibrinogène, du FXIII et de colle biologique sans risque de dénaturation, et, d'autre part, une solubilisation rapide des lyophilisats, typiquement en 3 à 8 min.

Dans un mode de réalisation préféré, le tampon de diafiltration contient le mélange A (comprenant un mélange de 10 à 12 g/l de citrate trisodique, de 1 à 5 g/l de lysine, de 1 à 5 g/l de glycine, de 2 à 5 g/l de Tris, de 25 à 50 g/l d'arginine et 5 à 15 g/l d'isoleucine), et est à pH 6,9-7,1.

Avantageusement, le tampon de diafiltration comprenant le mélange A est le même que celui qui est utilisé pour éluer la colle biologique de la résine d'échange d'anions. Dans ce mode de réalisation préféré, la mise en oeuvre de la diafiltration et du procédé de l'invention s'en trouvent donc simplifiés et optimisés.

Des tampons de diafiltration de composition différente peuvent aussi être utilisés en fonction des besoins, à condition qu'ils remplissent les critères énoncés ci-dessus. L'étape d'ultrafiltration mentionnée plus haut peut être également mise oeuvre dans les mêmes conditions à ce stade du procédé.

Les solutions diafiltrées, éventuellement concentrées par ultrafiltration, sont lyophilisées selon des méthodes classiques et conditions habituelles, à savoir entre -40°C et -30°C pendant environ 48 heures.

Dans un mode de réalisation préféré, le procédé de l'invention comprend au moins une étape de traitement d'inactivation virale et/ou d'élimination de virus et des contaminants tels que par exemple le prion. Ce traitement peut être choisi dans le groupe constitué par le traitement chimique d'inactivation virale; la nanofiltration et le traitement thermique d'inactivation virale à sec.

Lorsque le traitement d'inactivation virale est un traitement chimique, il correspond avantageusement à un traitement par solvant-détergent, suivant la méthode décrite dans le brevet EP 0 131 740. Les agents chimiques d'inactivation virale utilisés correspondent préférentiellement à un mélange de Tween-TnBP, et de façon plus préférée à un mélange de Triton (octoxinol)-TnBP, dont les concentrations typiques sont respectivement de 0,3% (v/v) et 1% (p/v). L'inactivation virale par traitement chimique peut être intégrée à un stade quelconque du procédé, mais elle est judicieusement mise en oeuvre avant l'étape h) de purification chromatographique. De cette façon, la purification chromatographique contribuera à l'élimination efficace des agents d'inactivation.

Dans un mode préféré de mise en oeuvre du procédé, il peut également être prévu une étape de nanofiltration pour éliminer les virus, notamment non enveloppés, et autres contaminants exogènes, ladite étape pouvant être utilisée en complément de l'étape de traitement chimique d'inactivation virale décrite ci-dessus. La nanofiltration peut avantageusement être mise en oeuvre sur des filtres d'une porosité de 35 nm et/ou de 15 nm, bien que d'autres filtres nanométriques puissent être utilisés dans la mesure où les durées de filtration et l'efficacité de rétention virale sont optimisées.

La nanofiltration est avantageusement effectuée soit sur l'éluat résultant de l'étape h) soit, le cas échéant, sur les solutions diafiltrées de fibrinogène, de colle biologique et/ou de FXIII remis en solution, avant lyophilisation de ces dernières.

Il convient de noter que la technique de nanofiltration requiert, pour une mise en oeuvre efficace, de maîtriser les paramètres physico-chimiques influant sur le rendement de récupération des composés à filtrer, et ce en évitant le colmatage du filtre et le passage de divers virus et contaminants. Ces paramètres, tels que la force ionique, le pH de la solution, ainsi que les conditions opératoires de la filtration, imposent des conditions spécifiques de mise en oeuvre qui dépendent également de la nature du ou des composés présents dans la solution à filtrer.

Le choix judicieux des paramètres chimiques de la purification chromatographique et de ceux de la diafiltration permet donc de mettre en oeuvre la nanofiltration sans en altérer les performances.

Enfin, un traitement thermique d'inactivation virale à sec est avantageusement effectué sur les lyophilisats de fibrinogène, de colle biologique et de FXIII obtenus après l'étape de lyophilisation, selon des conditions, classiques, à 80°C pendant 72 heures, pour inactiver des virus non enveloppés qui n'auraient pas été inactivés et/ou éliminés par au moins l'une des deux étapes d'inactivation virale et/ou d'élimination des virus décrites ci-dessus.

Les lyophilisats chauffés à sec peuvent ensuite être reconstitués dans un milieu aqueux compatible avec un usage clinique, de préférence dans de l'eau purifiée pour injection (PPI), et directement injectés par voie intraveineuse.

Ainsi, là mise en oeuvre du procédé conduit à des lyophilisats de concentrés de colle biologique et de fibrinogène hautement purifiés, dont la teneur respective en fibrinogène, par rapport à la teneur totale de protéines, est d'environ 85 à 90%.

Sans l'utilisation du disperseur en ligne, le teneur en fibrinogène, par rapport à la teneur totale de protéines est comprise entre 70 et 77 % (voir tableau A).

De plus, les activités de Facteur XIII dans les concentrés de colle biologique et de fibrinogène sont respectivement d'environ 5 U/ml et d'environ 1,5 U/ml.

Le concentré de Facteur XIII obtenu est dépourvu de protéines contaminantes et présente une activité, selon les besoins, située dans la plage de valeurs allant d'environ 30 U/ml à environ 700 U/ml, de préférence de 100 U/ml à 400 U/ml, obtenue en fonction de la concentration de remise en solution du précipité de FXIII et/ou de celle obtenue après ultrafiltration.

De plus, la présence d'au moins une étape de traitement d'inactivation virale et/ou d'élimination des virus et de contaminants dans le procédé de l'invention rend les concentrés de colle biologique, de fibrinogène et de facteur XIII susceptibles d'être obtenus par ce procédé appropriés à un usage thérapeutique.

L'exemple qui suit illustre un mode de réalisation de la présente invention sans toutefois en limiter la portée.

### Exemple

On utilise 1200 1 de plasma humain dépourvu de cryoprécipité. Ce plasma est soumis à une précipitation éthanolique par la méthode de Cohn, selon des conditions connues de l'homme du métier, de façon telle que la concentration d'éthanol dans le plasma considéré soit de 8% (v/v) et la température du mélange ainsi obtenu soit de -3°C à -5°C.

Le surnageant et le précipité ainsi obtenus sont ensuite centrifugés. On obtient entre 8 et 12 kg de précipité éthanolique, qui constitue la fraction I de Cohn impure.

La fraction I de Cohn impure est remise en suspension et lavée par 300 1 de tampon « Blombach » (Blombäck B, Blombäck M: Purification of human and bovine fibrinogen. Ark Kem 10:415-443, 1956) constitué d'un mélange de glycine 1 M, de citrate trisodique 0,055 M et d'éthanol 6,5% (v/v), à un pH de 6,8.

Le lavage est réalisé au moyen d'un système de dispersion en ligne de type Z66 (Ystral). Après centrifugation sur centrifugeuse Sharpless AS 16, avec des injecteurs calibrés à 3 mm, on récupère environ 8 kg de pâte plasmatique lavée (fraction I de Cohn purifiée) qui sont dissous, à une température de 37°C, dans 60 1 de tampon constitué d'un mélange de chlorure de sodium 0,12 M, de citrate trisodique 0,010 M et d'arginine 0,05 M, de pH 7,4. La pâte plasmatique solubilisée ainsi obtenue est ensuite soumise à un traitement par traitement au gel d'alumine, à raison de 108 g pour 1 kg de pâte plasmatique, à une température de 25°C et à un pH de 6,9-7,1.

Une fois le traitement au gel d'alumine effectué, la solution est soumise à des filtrations lenticulaires profondeur, par l'intermédiaire de filtres en fibres de cellulose (Seitz, type K700) de 0,65 micron m et stérilisantes par des filtres de 0,2 µm.

Cette solution est ensuite soumise à un premier traitement d'inactivation virale par solvant-détergent en présence de Tween-TnBP, de concentrations respectives de 0,3% (v/v) et 1% (p/v), selon la méthode décrite dans EP 0 131 740.

Le tableau A compare la concentration de fibrinogène, de protéines et le degré de pureté du fibrinogène obtenus lors de la mise en oeuvre d'un procédé comprenant une étape de lavage par dispersion en ligne (tel que celui décrit ci-dessus) avec ceux d'un procédé similaire dans lequel l'étape de lavage est réalisée en l'absence d'un système de dispersion en ligne. Il convient de noter que les résultats présentés dans le tableau A correspondent à des moyennes de valeurs obtenues respectivement à partir de 2 lots de plasma différents pour le procédé ne comprenant pas d'étape de lavage par dispersion et de 3 lots de plasmas différents pour le procédé comprenant une étape de lavage par dispersion. A l'exception de l'utilisation d'un disperseur en ligne de type rotor/stator lors du lavage du précipité éthanolique, les procédé décrit dans le tableau A sont strictement comparables (y compris pour l'ensemble des tampons et températures utilisés).

**Tableau A - Résultat de contrôles en cours de production**

| | | Procédé ne comprenant pas d'étape de lavage par dispersion | Procédé comprenant une étape de lavage par dispersion en ligne |
|---|---|---|---|
| | | Moyenne de 2 lots | Moyenne de 3 lots |
| Matière première plasmatique avant précipitation éthanolique | Volume (L) | 796 | 778 |
| | Protéines (g/L) | 56 | 54,83 |
| | Fibrinogène coagulable (g/L) | 1,55 | 1,46 |
| | Pureté Fibrinogène (%) | 2,76 | 2,66 |
| pâte plasmatique solubilisée | Volume (L) | 53 | 54,33 |
| | Protéines (g/L) | 21,25 | 17,83 |
| | Fibrinogène coagulable (g/L) | 15,15 | 15,9 |
| | Pureté Fibrinogène (%) | 71,29 | 89,17 |
| Surnageant obtenu après traitement de la pâte plasmatique solubilisée par l'hydroxyde d'aluminium | Volume (L) | 55 | 54 |
| | Protéines (g/L) | 16,5 | 15,16 |
| | Fibrinogène coagulable (g/L) | 11,85 | 12,96 |
| | Pureté Fibrinogène (%) | 71,81 | 85,13 |
| Fraction plasmatique soluble après filtration clarifinate et stérilisante | Volume (L) | 53,45 | 53,3 |
| | Protéines (g/L) | 16 | 14,33 |
| | Fibrinogène coagulable (g/L) | 12,35 | 12,53 |
| | Pureté Fibrinogène (%) | 77,18 | 87.43 |

Il apparaît clairement que les résultats obtenus avec le disperseur en ligne sont significativement supérieurs, dans la mesure où le degré de pureté du fibrinogène est toujours significativement plus élevé que celui obtenu avec le procédé utilisant un système de lavage classique. Il apparaît donc que l'efficacité du disperseur en ligne mis en place, a contribué à l'amélioration de l'étape de lavage et par voie de conséquence, à l'augmentation de la pureté du fibrinogène à ce stade du procédé. Le disperseur en ligne permet ainsi d'obtenir une solution plus homogène, avec une dispersion très fine du précipité éthanolique dans le tampon de lavage, et contribue de ce fait à l'élimination d'une proportion plus importante de contaminants. Il est en outre important de noter que le contrôle visuel de la solution ne met pas en évidence d'agglomérats susceptibles d'obturer les injecteurs des centrifugeuses dans les étapes suivant l'étape de lavage.

Par ailleurs, les concentrations de différentes protéines contaminantes ont été mesurées dans la fraction plasmatique soluble résultant de cette première partie du procédé. Ces résultats sont résumés dans le tableau B ci-dessous :

**Tableau B :**

| | | Procédé ne comprenant pas d'étape de lavage par dispersion | Procédé comprenant une étape de lavage par dispersion en ligne |
|---|---|---|---|
| | | Moyenne de 2 lots | Moyenne de 3 lots |
| Protéines totales | g/L | 16.00 | 14.33 |
| Protéines co-purifiées | | | |
| Fibronectine | mg/L | 746,75 | 607,8 |
| IgG | mg/L | 837,25 | 325,5 |
| IgA | mg/L | 92,35 | 83,81 |
| IgM | mn/L | 257,75 | 114,9 |
| C3c | g/L | 0,13 | 0,06 |
| C4 | g/L | 0,155 | 0,036 |
| Plasminogène | µg/L | 69,68 | 53,25 |
| FII | µU/L | 743,8 | 578,1 |
| **Protéines co-purifiées** | **g/L** | **2.362** | **1.289** |
| **Protéines co-purifiées** | **%** | **14.76** | **8.99** |

Il ressort clairement des résultats présentés dans le tableau B que l'étape de lavage par dispersion en ligne permet d'éliminer un nombre significativement plus élevé de protéines co-purifiées (protéines contaminantes) accompagnant le fibrinogène.

La fraction plasmatique soluble diluée est ensuite injectée sur une colonne chromatographique remplie d'un gel échangeur d'anions DEAE Macroprep (Bio-Rad, France), préalablement équilibrée en tampon constitué de chlorure de sodium 0,06 M et de citrate trisodique 0,011 M, ajusté à un pH de 8,0 M, d'osmolarité de 130-150 mosmolkg⁻¹. Dans ces conditions, le fibrinogène et le Facteur XIII, constituant la colle biologique, sont retenus par le support. Les protéines faiblement ou non retenues par le support sont éliminées dans le filtrat, ainsi que les Tween et TnBP, par plusieurs lavages successifs avec le même tampon.

Lorsque la DO, mesurée à 280 nm, est retombée à la valeur de celle de la ligne de base, l'élution du fibrinogène et du Facteur XIII, constituant la colle biologique, est effectuée par un tampon d'élution contenant du chlorure de sodium 1 M et un mélange A' constitué de citrate trisodique (11,2 g/1), de lysine (2,0 g/1), de glycine (2,0 g/1), le sel tris (2,40 g/1), d'arginine (40 g/1) et l'isoleucine (10 g/1), ajusté à un pH de 7,5, d'osmolarité > 2000 mosmolkg⁻¹

L'éluat de colle biologique purifiée ainsi récupéré est soumis à une nanofiltration sur des filtres PLANOVA (Asahi - Japon) de 35 nm et de 1m² de surface, afin d'éliminer les virus qui n'auraient pas été inactivés par le traitement solvant-détergent ci-dessus. La teneur en protéines totales à ce stade du procédé est d'environ 4,0 g/l de solution.

On isole 50% du volume de l'éluat de colle biologique et une solution de citrate trisodique 1 M est ajoutée au restant du volume d'éluat pour faire précipiter le Facteur XIII. Après s'être assuré que tout le Facteur XIII a précipité, celui-ci est isolé et récupéré par filtration sur dès filtres Sartopure (Sartorius - France) de 5µm.

Le précipité de FXIII ainsi récupéré est remis en solution dans de l'eau purifiée pour injection, à raison d'environ 1 g/1.

Les solutions de colle biologique (éluat) et de fibrinogène, sont concentrées par ultrafiltration sur des filtres Biomax Millipore 100 kDa de 5 m² de surface, de façon à ce que la teneur en protéines de chacune, des solutions atteigne 15 g/1.

Les solutions concentrées ci-dessus et la solution de FXIII sont soumises à une diafiltration sur les mêmes filtres que ceux utilisés pour l'ultrafiltration contre le mélange A' défini ci-dessus, de pH 6,9-7,1, d'osmolarité de 590-610 mosmolkg ⁻¹, ce qui permet également l'élimination du chlorure de sodium.

Après avoir procédé à une filtration stérilisante sur filtres de 0,45-0,2 micron m, on prélève 100 ml des solutions respectives diafiltrées, et on les place dans des flacons en verre en vue d'une lyophilisation mise en oeuvre entre -40°C et -30°C pendant environ 48 heures.

Les lyophilisats de fibrinogène, de colle biologique et de Facteur XIII obtenus sont soumis à une ultime étape d'inactivation virale par chauffage à sec à 80°C pendant 72 heures et stockés en vue d'une utilisation thérapeutique ultérieure.

## Revendications

1. Procédé de traitement du plasma sanguin comprenant les étapes de :
a) précipitation éthanolique du plasma ou d'une fraction de plasma ;
b) récupération du précipité formé à l'étape a);
c) lavage dudit précipité par dispersion qui est réalisé en utilisant un disperseur de type rotor/stator;
d) récupération d'une pâte plasmatique lavée ; et
e) solubilisation de ladite pâte plasmatique lavée.

2. Procédé de traitement du plasma sanguin selon la revendication 1 dans lequel le lavage par dispersion de l'étape c) est réalisé en utilisant un disperseur en ligne, le précipité de l'étape b) étant fourni à l'entrée axiale du disperseur et le produit lavé étant rejeté à la sortie radiale du disperseur.

3. Procédé de traitement du plasma sanguin selon la revendication 1 ou 2, dans lequel la récupération du précipité à l'étape b) et/ou la récupération de la pâte plasmatique à l'étape d) sont effectuées par centrifugation.

4. Procédé de traitement du plasma sanguin selon la revendication 3, dans lequel la récupération de la pâte plasmatique à l'étape d) est effectuée par la mise en oeuvre d'une centrifugeuse à injection.

5. Procédé de traitement du plasma sanguin selon l'une quelconque des revendications 1 à 4, dans lequel le produit lavé à l'étape c) est sensiblement homogène.

6. Procédé de traitement du plasma sanguin selon l'une quelconque des revendications 1 à 5, comprenant les étapes additionnelles de :
f) traitement de ladite pâte plasmatique solubilisée par l'hydroxyde d'aluminium et/ou traitement de ladite pâte plasmatique solubilisée à basse température ;
g) obtention d'une fraction plasmatique soluble par filtration clarifiante et/ou stérilisante du surnageant résultant de l'étape f).

7. Procédé de traitement du plasma sanguin selon l'une des revendications 1 à 6 dans lequel le lavage par dispersion de l'étape c) est réalisé avec un tampon constitué d'un mélange de glycine 0.5-1.5 M, de citrate trisodique 0.01-0.1 M et d'éthanol 5.0-8.0 % (v/v), à un pH de 6.7-6.9.

8. Procédé de traitement du plasma sanguin comprenant le procédé des revendications 1 à 7 et comprenant les étapes additionnelles de:
h) purification chromatographique de la fraction plasmatique soluble de l'étape g) sur une résine de type échangeur d'anions de type base faible préalablement équilibré par un tampon de force ionique prédéterminée de pH basique, dans des conditions permettant la rétention de la colle biologique, ladite colle biologique étant éluée par augmentation de la force ionique dudit tampon ;
i) précipitation du FXIII à partir de tout ou partie de l'éluat de colle biologique obtenu à l'étape h) par ajout d'au moins un agent chimique précipitant le FXIII ;
j) récupération d'une solution de fibrinogène purifié correspondant au surnageant de l'étape i) ; et
k) diafiltration des solutions de fibrinogène, et/ou de colle biologique et/ou de FXIII remis en solution , suivie d'une lyophilisation desdites solutions.

9. Procédé selon l'une quelconque des revendications 1 à 8, **caractérisé en ce qu'**il comprend au moins une étape de traitement d'inactivation virale et/ou d'élimination de virus et de contaminants, le traitement étant choisi dans le groupe constitué par le traitement chimique d'inactivation virale, la nanofiltration et le traitement thermique d'inactivation virale à sec.

10. Procédé selon l'une quelconque des revendications 6 à 9, **caractérisé en ce qu'**il comprend une étape d'ultrafiltration de concentration effectuée préalablement à l'étape de diafiltration k) ou postérieurement à ladite étape, avant lyophilisation.

## Patentansprüche

1. Verfahren zur Behandlung von Blutplasma, umfassend die folgenden Schritte:
a) ethanolische Ausfällung des Plasmas oder der Plasmafraktion;
b) Gewinnung der in Schritt a) gewonnenen Ausfällung;
c) Waschen der Ausfällung durch Dispersion, welche unter Verwendung eines Dispergierers vom Typ Rotor/Stator durchgeführt wird;
d) Gewinnung einer gewaschenen Plasmamasse; und
e) Verflüssigung der gewaschenen Plasmamasse.

2. Verfahren zur Behandlung von Blutplasma nach Anspruch 1, bei welchem das Waschen durch Dispersion in Schritt c) unter Verwendung eines Inline-Dispergierers erfolgt, wobei die Ausfällung aus Schritt b) dem axialen Eingang des Dispergierers zugeführt wird und das gewaschene Produkt am radialen Ausgang des Dispergierers ausgeworfen wird.

3. Verfahren zur Behandlung von Blutplasma nach Anspruch 1 oder 2, bei welchem die Gewinnung der Ausfällung aus Schritt b) und/oder die Gewinnung der Plasmamasse aus Schritt d) durch Zentrifugieren erfolgen.

4. Verfahren zur Behandlung von Blutplasma nach Anspruch 3, bei welchem die Gewinnung der Plasmamasse aus Schritt d) durch Anwendung einer Einspritzzentrifuge erfolgt.

5. Verfahren zur Behandlung von Blutplasma nach einem der Ansprüche 1 bis 4, bei welchem das gewaschene Produkt aus Schritt c) im Wesentlichen homogen ist.

6. Verfahren zur Behandlung von Blutplasma nach einem der Ansprüche 1 bis 5, welches folgende zusätzliche Schritte umfasst:
f) Behandlung der verflüssigten Plasmamasse mit Aluminiumhydroxid und/oder Behandlung der verflüssigten Plasmamasse bei niedriger Temperatur;
g) Erhalts einer löslichen Plasmafraktion durch klärende und/oder sterilisierende Filtration des Überstands aus Schritt f).

7. Verfahren zur Behandlung von Blutplasma nach einem der Ansprüche 1 bis 6, bei welchem das Waschen durch Dispersion aus Schritt c) mit einem Tampon erfolgt, der eine Mischung aus 0,5-1,5 M Glycin, 0,01-0,1 M Trinatriumcitrat und 5,0-8,0 % (v/v) Ethanol umfasst, mit einem pH-Wert von 6,7-6,9.

8. Verfahren zur Behandlung von Blutplasma, welches das Verfahren nach den Ansprüchen 1 bis 7 sowie die folgenden zusätzlichen Schritte umfasst:
h) chromatographische Reinigung der löslichen Plasmafraktion aus Schritt g) auf einem Harz vom Typ Anionenaustauscher vom Typ schwach basisch, vorangehend ausgeglichen durch einen Tampon mit vordefinierter Ionenstärke mit basischem pH-Wert, unter Bedingungen, die das Rückhalten des biologischen Klebers erlauben, wobei der biologische Kleber durch Erhöhung der Ionenstärke des Tampons eluiert wird;
i) Ausfällung des FXIII ausgehend vom gesamten Eluat oder einem Teil des Eluats des in Schritt h) erhaltenen biologischen Klebers durch Zufügen wenigstens einer chemischen Substanz zur Ausfällung des FXIII;
j) Gewinnung einer gereinigten Fibrinogenlösung entsprechend dem Überstand aus Schritt i); und
k) Diafiltration der Lösungen aus Fibrinogen und/oder biologischem Kleber und/oder FXIII in Lösung, gefolgt von einem Lyophilisieren dieser Lösungen.

9. Verfahren nach einem der Ansprüche 1 bis 8, **dadurch gekennzeichnet dass** es wenigstens einen Behandlungsschritt der Virusinaktivierung und/oder Schritt zur Entfernung von Viren und Verunreinigungen umfasst, wobei die Behandlung aus der Gruppe, umfassend eine chemische Behandlung zur Virusinaktivierung, eine Nanofiltration und eine trockene Hitzebehandlung zur Virusinaktivierung gewählt wird.

10. Verfahren nach einem der Ansprüche 6 bis 9, **dadurch gekennzeichnet dass** es einen Schritt der Konzentrierung durch Ultrafiltration umfasst, der vor dem Schritt der Diafiltration k) oder nach diesem Schritt vor dem Lyophilisieren durchgeführt wird.

## Claims

1. Method for treating blood plasma comprising the steps of:
a) ethanol precipitation of the plasma or a plasma fraction;
b) retrieval of the precipitate formed in step a);
c) washing of said precipitate by dispersion which is carried out using a rotor/stator type disperser;
d) retrieval of a washed plasma paste; and
e) solubilisation of said washed plasma paste.

2. Method for treating blood plasma according to claim 1 wherein the washing by dispersion in step c) is carried out using an inline disperser, the precipitate from step b) being supplied at the axial inlet of the disperser and the washed product being discharged at the radial outlet of the disperser.

3. Method for treating blood plasma according to claim 1 or 2, wherein the retrieval of the precipitate in step b) and/or the retrieval of the plasma paste in step d) are performed by centrifugation.

4. Method for treating blood plasma according to claim 3, wherein the retrieval of the plasma paste in step d) is performed using an injection centrifuge.

5. Method for treating blood plasma according to any one of claims 1 to 4, wherein the product washed in step c) is substantially homogeneous.

6. Method for treating blood plasma according to any one of claims 1 to 5, comprising the additional steps of:
f) treatment of said solubilised plasma paste with aluminium hydroxide and/or treatment of said solubilised plasma paste at a low temperature;
g) preparation of a soluble plasma fraction by clarifying and/or sterilising filtration of the supernatant resulting from step f).

7. Method for treating blood plasma according to any one of claims 1 to 6 wherein the washing by dispersion in step c) is carried out with a buffer consisting of a mixture of 0.5-1.5 M glycine, 0.01-0.1 M trisodium citrate and 5.0-8.0% (v/v) ethanol, at a pH of 6.7-6.9.

8. Method for treating blood plasma comprising the method according to claims 1 to 7 and comprising the additional steps of:
h) chromatographic purification of the soluble plasma fraction from step g) on a weak base type anion exchanger type resin previously equilibrated with a buffer of predetermined ionic strength having a basic pH, under conditions enabling the retention of the bioadhesive, said bioadhesive being eluted by increasing the ionic strength of said buffer;
i) precipitation of FXIII using all or part of the bioadhesive eluate obtained in step h) by adding at least one chemical agent precipitating FXIII;
j) retrieval of a purified fibrinogen solution corresponding to the supernatant from step i); and
k) diafiltration of the resolubilised fibrinogen, and/or bioadhesive and/or FXIII solutions, followed by freeze-drying of said solutions.

9. Method according to any one of claims 1 to 8, **characterised in that** it comprises at least one step for viral inactivation treatment and/or removing viruses and contaminants, the treatment being chosen in the group consisting of chemical viral inactivation treatment, nanofiltration and dry viral inactivation heat treatment.

10. Method according to any one of claims 6 to 9, **characterised in that** it comprises a concentration ultrafiltration step performed prior to the diafiltration step k) or after said step, before freeze-drying.
